# EUROPEAN PATENT APPLICATION

(11) **EP 4 006 697 A1**
(43) Date of publication of application: **01.06.2022**
(21) Application number: 19954831.4
(22) Date of filing: 05.12.2019
(51) Int. Cl.: G06F 3/01

(54) **CONTROL DEVICE, CONTROL METHOD, AND PROGRAM**

(71) Applicant: Japan Tobacco Inc., Tokyo 105-6927 (JP)
(72) Inventor: SUGANO, Yuka, Tokyo 130-8603 (JP); SERITA, Kazutoshi, Tokyo 130-8603 (JP); SENJU, Masatoshi, Tokyo 130-8603 (JP)
(74) Representative: Hoffmann Eitle
(86) International application number: PCT/JP2019/047610
(87) International publication number: WO 2021/111578

(57) **Abstract**

[Problem] To provide a mechanism with which it is possible to relax and enjoy an inhalation experience.

[Solution] A control device provided with a control unit which interrupts the progress of an application being executed on a terminal device if an interrupting condition relating to an inhalation device associated with the terminal device is satisfied.

## Description

### Technical Field

The present invention relates to a control device, a control method, and a program.

### Background Art

Inhaler devices, such as electronic cigarettes and nebulizers, generating material to be inhaled by users are in widespread use. For example, an inhaler device uses an aerosol source for generating an aerosol and a substrate including a flavor source for imparting a flavor component to the generated aerosol to generate the aerosol to which the flavor component is imparted. When a user inhales the aerosol, generated by the inhaler device, to which the flavor component is imparted (hereinafter, also referred to as puffing), the user can enjoy the flavor.

In recent years, techniques in which an inhaler device and another device such as a smartphone are wirelessly connected to each other so as to be in corporation with each other to offer users a new inhalation experience have been studied. For example, Patent Literature 1 listed below discloses a technique in which an avatar is displayed on a smartphone on the basis of information received from an inhaler device and a profile of the avatar received from a cloud server to enable communication between users via the avatar.

### Citation List

### Patent Literature

Patent Literature 1: JP2018-536309A

### Summary of Invention

### Technical Problem

With the technique described in Patent Literature 1 listed above, however, users may be distracted by an application in progress and may have difficulty in enjoying an inhalation experience in a concentrated manner.

Accordingly, the present invention has been made in view of the above-described issue, and an object of the present invention is to provide a system for allowing users to enjoy an inhalation experience in a concentrated manner.

### Solution to Problem

In order to solve the above-described issue, according to an aspect of the present invention, there is provided a control device including: a controller that suspends an application in progress, in response to a condition for suspension related to an inhaler device associated with a terminal device being satisfied while the application is being executed by the terminal device.

The condition for suspension may include a condition that a predetermined user action performed by using the inhaler device is detected.

The predetermined user action may be inhalation using the inhaler device.

The predetermined user operation may be an operation performed on the inhaler device for enabling inhalation using the inhaler device.

The condition for suspension may further include a condition that a predetermined event occurs in the application.

The predetermined event may include an event for which an operation is to be performed on the application within a predetermined time from when the event occurs.

The controller may resume the suspended application in response to a condition for resumption related to the inhaler device being satisfied.

The condition for resumption may include a condition that an elapsed time since detection of the predetermined user action performed by using the inhaler device reaches a first threshold.

The controller may set the predetermined threshold on the basis of a characteristic of a user of the inhaler device.

The controller may set the predetermined threshold on the basis of a type of the detected predetermined user action.

The controller may set the predetermined threshold on the basis of the number of times the predetermined user action performed is detected while the application is suspended.

The condition for resumption may include a condition that the number of times the predetermined user action performed is detected while the application is suspended reaches a second threshold.

The controller may provide to a user of the terminal device, information indicating a timing when the suspended application is to be resumed.

The terminal device may include a display unit that displays an output screen of the application, and the controller may control display by the display unit while the application is suspended, on the basis of a characteristic of the application.

For the application, a first action mode in which suspension of the application is allowed or a second action mode in which suspension of the application is not allowed may be set, and the condition for suspension may further include a condition that the first action mode is set for the application.

The control device may be included in the inhaler device.

The control device may be included in the terminal device.

In order to solve the above-described issue, according to another aspect of the present invention, there is provided a control method including: suspending an application in progress, in response to a condition for suspension related to an inhaler device associated with a terminal device being satisfied while the application is being executed by the terminal device.

In order to solve the above-described issue, according to another aspect of the present invention, there is provided a program for causing a computer to function as: a controller that suspends an application in progress, in response to a condition for suspension related to an inhaler device associated with a terminal device being satisfied while the application is being executed by the terminal device.

### Advantageous Effects of Invention

As described above, according to the present invention, a system for allowing users to enjoy an inhalation experience in a concentrated manner is provided.

### Brief Description of Drawings

[Fig. 1] Fig. 1 is a schematic diagram of an inhaler device according to a first configuration example.
[Fig. 2] Fig. 2 is a schematic diagram of an inhaler device according to a second configuration example.
[Fig. 3] Fig. 3 is a block diagram of an example of the configuration of a system according to one embodiment of the present invention.
[Fig. 4] Fig. 4 is a schematic diagram of operations of a terminal device in a first specific example.
[Fig. 5] Fig. 5 is a flowchart of an example of the flow of a process, in the first specific example, performed by the terminal device according to the present embodiment.
[Fig. 6] Fig. 6 is a schematic diagram of operations of the terminal device in a second specific example.
[Fig. 7] Fig. 7 is a flowchart of an example of the flow of a process, in the second specific example, performed by the terminal device according to the present embodiment.

### Description of Embodiments

Hereinafter, a preferred embodiment of the present invention will be described in detail with reference to the attached drawings. Note that in the specification and drawings, structural elements having substantially the same functional configurations are assigned the same reference signs to thereby omit a duplicated description.

### 1. Configuration example of inhaler device

An inhaler device generates material to be inhaled by a user. In the example described below, the material generated by the inhaler device is an aerosol. Alternatively, the material generated by the inhaler device may be gas.

### (1) First configuration example

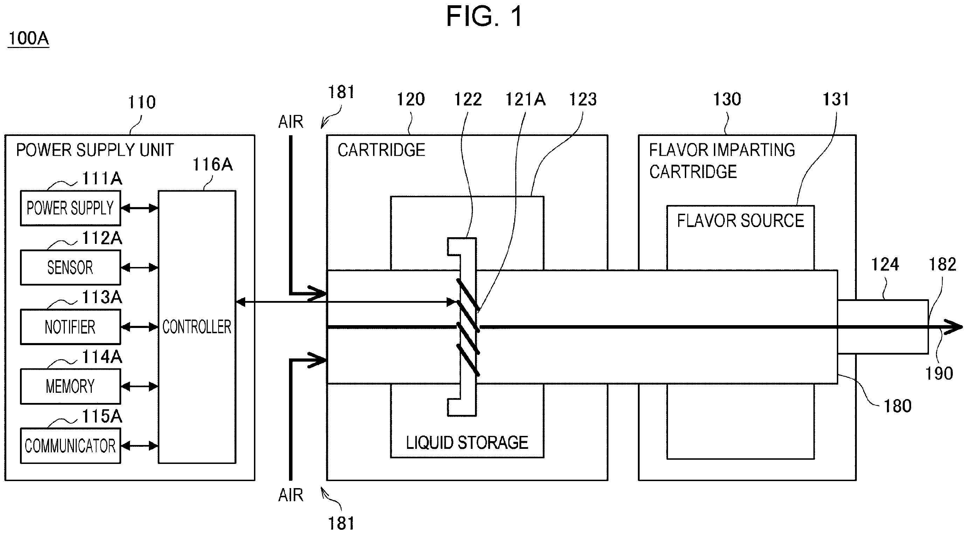

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a microphone condenser, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark) or Bluetooth (registered trademark).

The controller 116A functions as an arithmetic processing unit and a control circuit, and controls the overall operations of the inhaler device 100A in accordance with various programs. The controller 116A includes an electronic circuit such as a central processing unit (CPU) or a microprocessor, for example.

The liquid storage 123 stores an aerosol source. The aerosol source is atomized to generate an aerosol. The aerosol source is a liquid such as polyhydric alcohol or water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In addition, means for atomizing the aerosol source is not limited to heating by the heater 121A. For example, the means for atomizing the aerosol source may be vibration atomization or induction heating.

### (2) Second configuration example

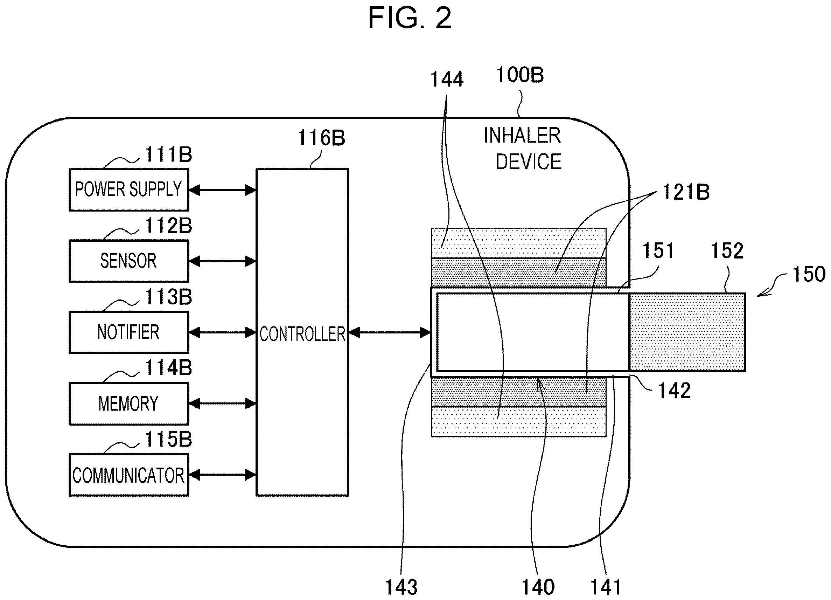

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 on its ends, and may define the pillar-shaped internal space 141. The holder 140 can also define a flow path of air to be supplied to the stick substrate 150. For example, the bottom 143 has an air inlet hole that is an inlet of air into the flow path. The opening 142 serves as an air outlet hole that is an outlet of the air from the flow path.

The stick substrate 150 includes a substrate 151 and an inhalation port 152. The substrate 151 includes an aerosol source. The aerosol source according to the present configuration example is not limited to a liquid. The aerosol source may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the air inlet hole (not illustrated), and the air and an aerosol generated from the substrate 151 reach inside the mouth of the user.

The heater 121B has a configuration the same as that of the heater 121A according to the first configuration example. However, in the example illustrated in Fig. 2, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also serve as an air inlet hole to the internal space 141. In this case, a mixture fluid of air and an aerosol generated by the heater 121A flows into the internal space 141, mixes further with an aerosol generated by the heater 121B, and then reaches the oral cavity of the user.

### 2. One embodiment

### 2.1 Configuration example

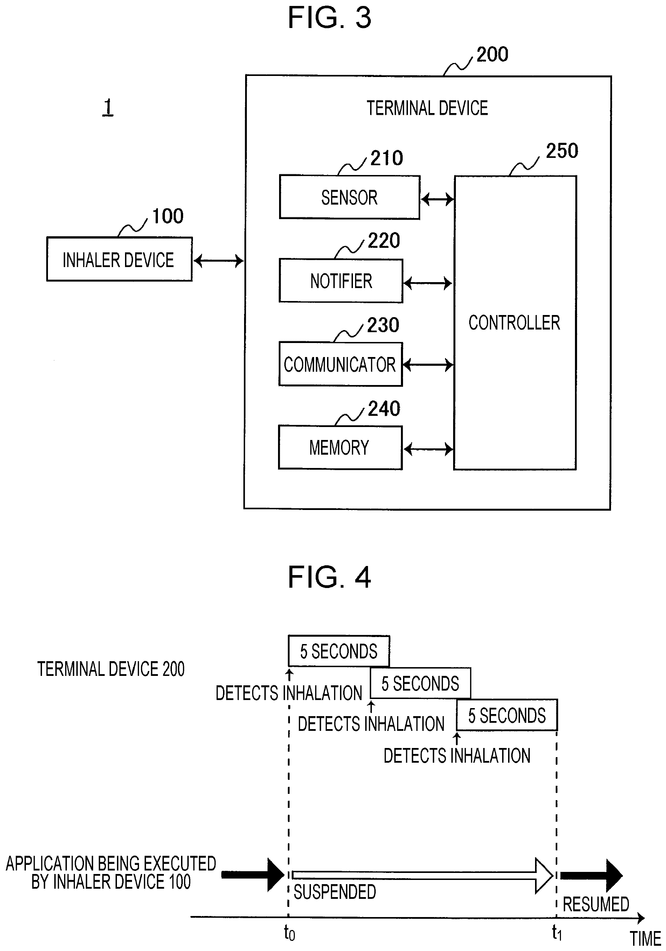

Fig. 3 is a block diagram of an example of the configuration of a system 1 according to one embodiment of the present invention. As illustrated in Fig. 3, the system 1 includes an inhaler device 100 and a terminal device 200.

The inhaler device 100 and the terminal device 200 are associated with each other. For example, the inhaler device 100 and the terminal device 200 are associated with each other in that the devices are used by the same user. For example, the inhaler device 100 and the terminal device 200 are associated with each other in that the devices are connected with each other in advance by, for example, pairing for wireless communication.

### (1) Inhaler device

The inhaler device 100 generates material to be inhaled by a user. In the present embodiment, the inhaler device 100 may have any of the first configuration example or the second configuration example described above. That is, the inhaler device 100 according to the present embodiment has a configuration the same as that of the inhaler device 100A or the inhaler device 100B or a modification of any of these configuration examples.

A description is given below of matters, of the configuration of the inhaler device 100 according to the present embodiment, to be supplemented or highlighted with respect to the configurations of the inhaler device 100A and the inhaler device 100B described in the above configuration examples.

A controller 116 according to the present embodiment controls a communicator 115 to transmit to the terminal device 200 various items of information for operations of the terminal device 200.

### (2) Terminal device

The terminal device 200 is an information processing device that is operated by a user. The terminal device 200 executes various applications. For example, the terminal device 200 may be a smartphone, a tablet terminal, or a wearable device.

As illustrated in Fig. 3, the terminal device 200 includes a sensor 210, a notifier 220, a communicator 230, a memory 240, and a controller 250.

The sensor 210 detects various items of information regarding the terminal device 200. The sensor 210 outputs the detected information to the controller 250. The sensor 210 includes an input unit that receives information input by the user. The input unit includes, for example, at least any of a button, a keyboard, a touch panel, or a microphone. The sensor 210 includes a positional information acquisition unit that acquires positional information indicating the position of the terminal device 200. The positional information acquisition unit receives a Global Navigation Satellite System (GNSS) signal from a GNSS satellite (for example, a Global Positioning System (GPS) signal from a GPS satellite) and detects positional information including the latitude and longitude of the device.

The notifier 220 provides information to the user. The notifier 220 includes at least any of a display device that displays information, a light-emitting device that emits light, a vibration device that vibrates, or a sound output device that outputs sound. The display device is, for example, a display. The light-emitting device is, for example, an LED. The vibration device is, for example, an eccentric motor. The sound output device is, for example, a speaker. The notifier 220 outputs information input from the controller 250 to thereby provide the information to the user. For example, the notifier 220 displays information to be provided to the user, emits light in a light-emission pattern corresponding to information to be provided to the user, vibrates in a vibration pattern corresponding to information to be provided to the user, or outputs information to be provided to the user by sound. The display device is an example of a display unit according to the present invention that displays an output screen of an application executed by the terminal device 200.

The communicator 230 is a communication interface for transmitting and receiving information between the terminal device 200 and another device. The communicator 230 performs communication in conformity with any wired or wireless communication standard. Such a communication standard may be, for example, a wireless local area network (LAN), a wired LAN, Wi-Fi (registered trademark) or Bluetooth (registered trademark). Typically, the communicator 230 wirelessly transmits and receives information to and from the inhaler device 100.

The memory 240 stores various items of information for operations of the terminal device 200. The memory 240 may be a nonvolatile storage medium such as flash memory.

The controller 250 functions as an arithmetic processing unit and a control device, and controls the overall operations of the terminal device 200 in accordance with various programs. The controller 250 includes, for example, an electronic circuit such as a central processing unit (CPU) or a microprocessor. In addition, the controller 250 may include a read-only memory (ROM) that stores, for example, a program and arithmetic parameters to be used and a random access memory (RAM) that temporarily sores, for example, parameters that change as appropriate. The terminal device 200 performs various processes under the control of the controller 250. Examples of the processes controlled by the controller 250 include processing of information detected by the sensor 210, provision of information by the notifier 220, transmission and reception of information by the communicator 230, and storing and reading of information in and from the memory 240. Other processes that are performed by the terminal device 200, such as input of information to the structural elements and processes based on information output from the structural elements, are also controlled by the controller 250.

### 2.2 Technical features

### (1) Overview

The terminal device 200 acquires inhalation information. The inhalation information is information regarding inhalation performed by a user using the inhaler device 100. The inhalation information is acquired by at least any of the inhaler device 100 or the terminal device 200. The inhalation information can be regarded as a use history of the inhaler device 100.

The inhalation information may be detected by a sensor 112. In an example, the inhalation information detected by the sensor 112 is information indicating whether the user has performed inhalation using the inhaler device 100. In another example, the inhalation information detected by the sensor 112 is information indicating whether a button included in the inhaler device 100 for giving an instruction for starting aerosol generation has been pressed. In response to pressing of the button included in the inhaler device 100 for giving an instruction for starting aerosol generation, for example, a heater 121 starts heating, and generation of an aerosol is started accordingly.

The inhalation information may be detected by the sensor 210. In an example, the inhalation information detected by the sensor 210 is positional information indicating the position of the terminal device 200 at the timing when the user performs inhalation or at the timing when the button for giving an instruction for starting aerosol generation is pressed.

The terminal device 200 performs various types of control on the basis of the acquired inhalation information. For example, the terminal device 200 controls the progress of an application that is being executed by the terminal device 200, on the basis of the acquired inhalation information. The terminal device 200 is an example of the control device according to the present invention.

In response to a condition for suspension related to the inhaler device 100 being satisfied while an application is being executed by the terminal device 200, the terminal device 200 suspends the application in progress. Specifically, the terminal device 200 suspends the application in progress, in response to a condition for suspension being satisfied, and maintains the state where the application is in progress, in response to a failure to satisfy the condition for suspension. The condition for suspension will be described in detail below. With such a configuration, the application in progress is suspended in response to the condition for suspension being satisfied, and the user can enjoy an inhalation experience in a concentrated manner without being distracted by the application.

In response to a condition for resumption related to the inhaler device 100 being satisfied, the terminal device 200 resumes the suspended application. Specifically, the terminal device 200 resumes the suspended application in response to a condition for resumption being satisfied, and maintains the state where the application is suspended, in response to a failure to satisfy the condition for resumption. The condition for resumption will be described in detail below. With such a configuration, the suspended application is automatically resumed in response to the condition for resumption being satisfied, and the user's convenience is increased.

Any application that is being executed by the terminal device 200 may be a target to be suspended and resumed. For example, various applications including a game, a phone call, a Social Networking Service (SNS), and an email may be targets to be suspended and resumed.

Suspending a game in progress means suspending the progress of time in the game. Resuming a game means resuming the game from the time when the game was suspended such that time progresses.

Suspending a phone call in progress means, for example, not notifying the user of an incoming call by, for example, not making a ringtone. Resuming a phone call means, for example, notifying the user of an incoming call that arrives during the period of suspension.

Suspending an SNS in progress means, for example, not notifying the user of information sent from the SNS by, for example, hiding the information. Resuming an SNS means, for example, notifying the user of information sent from the SNS during the period of suspension.

Suspending an email in progress means, for example, not notifying the user of an incoming email by, for example, hiding the incoming email. Resuming an email means, for example, notifying the user of an incoming email that arrives during the period of suspension.

A phone call in progress, an SNS in progress, and an email in progress may be suspended and resumed by disconnecting the terminal device 200 from the network, that is, switching to an airplane mode, and canceling the airplane mode.

### (2) Condition for suspension

### - Detection of inhalation-related action

The condition for suspension includes a condition that a predetermined user action performed by using the inhaler device 100 is detected.

The predetermined user action may be inhalation using the inhaler device 100. For example, in response to detection, by a pressure sensor, a flow sensor, or a temperature sensor included in the sensor 112, of a value generated in accordance with the user's inhalation, inhalation performed by using the inhaler device 100 is detected.

The predetermined user action may be an operation performed on the inhaler device 100 for enabling inhalation using the inhaler device 100. A state where inhalation using the inhaler device 100 is enabled is, for example, a state where the temperature of the stick substrate 150 reaches a predetermined temperature that enables inhalation, in the inhaler device 100B according to the second configuration example. Heating performed until the temperature of the stick substrate 150 reaches the predetermined temperature that enables inhalation is hereinafter also referred to as preheating. The operation performed on the inhaler device 100 for enabling inhalation using the inhaler device 100 is, for example, pressing of the button included in the sensor 112 for giving an instruction for starting aerosol generation.

In addition, the predetermined user operation may be detaching of the inhaler device 100 from a charging stand to or on which, for example, the inhaler device 100 is connected or placed in order to charge the inhaler device 100. The predetermined user operation may be turning on of the power of the inhaler device 100. Other various user operations may be detected as the predetermined user operations related to the condition for suspension.

The predetermined user action described above is hereinafter also referred to as an inhalation-related action. When the condition for suspension includes a condition that the inhalation-related action performed is detected, an application in progress can be suspended at the timing when the user starts or is to start inhalation using the inhaler device 100.

### - Occurrence of specific event

The condition for suspension may further include a condition that a predetermined event occurs in the application. In this case, the terminal device 200 suspends the application in progress when the inhalation-related action is detected and a predetermined event occurs in the application. When the inhalation-related action is detected but a predetermined event does not occur in the application, the terminal device 200 maintains the state where the application is in progress. The predetermined event is an event that may hinder the user from enjoying an inhalation experience in a concentrated manner. The predetermined event is hereinafter also referred to as a specific event. With such a configuration, the state where an application is in progress can be maintained as long as an event that may hinder the user from enjoying an inhalation experience in a concentrated manner does not occur in the application. In other words, with such a configuration, it is possible to keep the application running until an event that may hinder the user from enjoying an inhalation experience in a concentrated manner occurs.

The specific event may include an event for which an operation is to be performed on the application within a predetermined time from its occurrence. When the application is a game, the specific event is, for example, an event that brings a benefit to the user when the user performs an operation within a predetermined time from the occurrence or that brings a drawback to the user when the user does not perform an operation within the predetermined time from the occurrence. With such a configuration, when an event that brings a benefit or a drawback to the user occurs, it is possible to prevent the user from missing a chance to operate. In addition, with such a configuration, it is possible to keep the application running until an event that brings a benefit or a drawback to the user occurs.

### (3) Condition for resumption

The condition for resumption may include a condition that the elapsed time since detection of the inhalation-related action performed reaches a predetermined threshold (hereinafter also referred to as a first threshold). As the first threshold, a predicted value of the time from when the inhalation-related action is performed to when inhalation ends may be set. The end of inhalation means the user finishing inhalation. Alternatively, as the first threshold, a value obtained by adding a predicted value of the time taken for the user to enjoy the afterglow of inhalation to the predicted value of the time from when the inhalation-related action is performed to when inhalation ends may be set. The terminal device 200 measures the elapsed time since detection of the inhalation-related action performed and resumes the application in response to the measured elapsed time reaching the first threshold. When the inhalation-related action is performed again during measurement, the terminal device 200 resets the elapsed time that is being measured to zero and starts measurement again. With such a configuration, the application can be automatically resumed after the user finishes inhalation or after the user finishes enjoying the afterglow of inhalation, and the user's convenience can be increased.

The terminal device 200 may set the first threshold on the basis of the characteristics of the user. The characteristics of the user are characteristics that may affect the time taken to finish inhalation after the inhalation-related action is performed and the time taken to enjoy the afterglow of inhalation. In an example, the characteristics of the user include a past actual measurement value of the time taken to finish inhalation after the inhalation-related action is performed and a past actual measurement value of the time taken to enjoy the afterglow of inhalation. In another example, the characteristics of the user include attribute information, such as the age and sex, of the user, the time when inhalation is performed, and the place where inhalation is performed. The time taken to finish inhalation after the inhalation-related action is performed and the time taken to enjoy the afterglow of inhalation may vary between individuals. In this regard, with such a configuration, the application can be resumed at a timing more suitable to each individual user, and the user's convenience can be increased.

In addition, the terminal device 200 may set the first threshold on the basis of the type of the detected inhalation-related action. Specifically, the first threshold may be set to different values when inhalation is detected and when pressing of the button for giving an instruction for starting aerosol generation is detected. For example, when pressing of the button is detected, the terminal device 200 may set the first threshold to a value higher than a value set when inhalation is detected. This is because it takes time to finish preheating after pressing of the button is detected in the inhaler device 100B according to the second configuration example. With such a configuration, it is possible to prevent the application from being resumed during preheating.

In addition, the terminal device 200 may set the first threshold on the basis of the number of times the inhalation-related action performed is detected while the application is suspended. For example, the terminal device 200 may set the first threshold to a high value until the number of times inhalation is performed during the period of suspension reaches an expected number of times the user performs inhalation at once, and set the first threshold to a low value after the number of times inhalation is performed during the period of suspension reaches the expected number of times. With such a configuration, it is possible to prevent the application from being resumed while the user is performing inhalation a number of times.

The condition for resumption may include a condition that the number of times the inhalation-related action performed is detected while the application is suspended reaches a predetermined threshold (hereinafter also referred to as a second threshold). As the second threshold, for example, an expected number of times the user performs inhalation at once is set. The terminal device 200 counts the number of times the inhalation-related action is performed and resumes the application in response to the counted number reaching the second threshold. With such a configuration, the application can be automatically resumed after the user finishes inhalation performed a number of times, and the user's convenience can be increased. The second threshold may be set in accordance with the characteristics of the user similarly to the first threshold.

The terminal device 200 may provide to the user, information indicating the timing when the suspended application is to be resumed. In an example, the information indicating the timing when the suspended application is to be resumed is the remaining time before resumption, which is obtained by subtracting the elapsed time since detection of the inhalation-related action performed from the first threshold. In this case, for example, "the application will be resumed in 5 seconds" may be displayed. In another example, the information indicating the timing when the suspended application is to be resumed is the remaining number of times of inhalation before resumption, which is obtained by subtracting the number of times inhalation (that is, the inhalation-related action) performed is detected (hereinafter also referred to as the number of times of inhalation) from the second threshold. In this case, for example, "the number of times of puffing before resumption: 5" may be displayed. With such a configuration, the user can easily recognize the timing when the suspended application is to be resumed.

### (4) Others

While an application is suspended, the terminal device 200 may control display by the notifier 220 (which is a display device here) on the basis of the characteristics of the application. The characteristics of the application include, for example, the level of real-time property. When the level of real-time property is high, it can be regarded that the time before an operation is to be performed after resumption is short. For example, a shooting game is an application having a high level of real-time property. When the level of real-time property is low, it can be regarded that the time before an operation is to be performed after resumption is short. For example, a role-playing game in which a character does not act until a command is input is an application having a low level of real-time property. For example, for an application having a low level of real-time property, the terminal device 200 turns off the screen (that is, does not display the screen) while the application is suspended. When the level of real-time property is low, the user can have sufficient time to grasp the state shown on the screen after the screen is turned on upon resumption, and therefore, power consumption of the terminal device 200 can be suppressed without compromising the user's convenience. On the other hand, for an application having a high level of real-time property, when the screen is turned off during the period of suspension and subsequently turned on upon resumption, the user has difficulty in having sufficient time to grasp the state shown on the screen. Accordingly, for an application having a high level of real-time property, the terminal device 200 leaves the screen turned on (that is, leaves the screen displayed) while the application is suspended. The screen is left turned on during the period of suspension, and therefore, the user's convenience that may be compromised when the screen is turned off during the period of suspension can be maintained.

For an application, a first action mode in which suspension of the application is allowed or a second action mode in which suspension of the application is not allowed may be set. The terminal device 200 suspends/resumes an application that is executed in the first action mode on the basis of inhalation information. That is, the condition for suspension and the condition for resumption further include a condition that the first action mode is set for the application. On the other hand, the terminal device 200 does not suspend an application that is executed in the second action mode, at least on the basis of inhalation information. The action mode may be set by a user operation performed on the terminal device 200. Alternatively, the action mode may be set by a user operation performed on the inhaler device 100.

### (5) First specific example

A description of a process in a first specific example will be given below. In the first specific example, the condition for suspension is the condition that inhalation by the user is detected, and the condition for resumption is the condition that the elapsed time since detection of inhalation performed by the user reaches the first threshold. It is assumed that the first threshold is set to five seconds. It is assumed that the inhaler device 100 is the inhaler device 100A according to the first configuration example and preheating is not necessary.

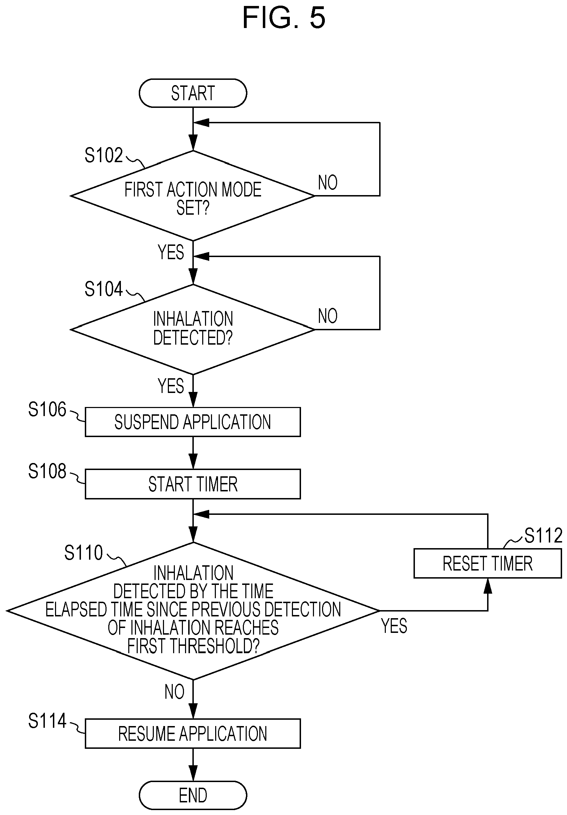

Fig. 4 is a schematic diagram of operations of the terminal device 200 in the first specific example. In the example illustrated in Fig. 4, first inhalation is detected at time to, second inhalation is detected within five seconds of the first inhalation, and third inhalation is detected within five seconds of the second inhalation. Thereafter, no inhalation is detected until time ti five seconds after detection of the third inhalation. Therefore, the terminal device 200 suspends an application that is being executed at time to and resumes the application at time ti. A description of the flow of the process in the present specific example will be given below with reference to Fig. 5.

Fig. 5 is a flowchart of an example of the flow of the process, in the first specific example, performed by the terminal device 200 according to the present embodiment. As illustrated in Fig. 5, the controller 250 determines whether the first action mode is set as the action mode of an application that is being executed (step S102). If it is determined that the first action mode is not set (NO in step S102), the process returns to step S102 again.

If it is determined that the first action mode is set (YES in step S102), the controller 250 determines whether inhalation by the user is detected (step S104). If it is determined that inhalation by the user is not detected (NO in step S104), the process returns to step S104 again. If it is determined that inhalation by the user is detected (YES in step S104), the controller 250 suspends the application that is being executed (step S106).

Next, the controller 250 starts a timer for measuring the elapsed time (step S108). Next, the controller 250 determines whether inhalation by the user is detected by the time the elapsed time since the previous detection of inhalation measured by the timer reaches the first threshold (step S110). If it is determined that inhalation by the user is detected by the time the elapsed time reaches the first threshold (YES in step S110), the controller 250 resets the timer to zero and measures the elapsed time again (step S112). On the other hand, if it is determined that inhalation by the user is not detected by the time the elapsed time reaches the first threshold (NO in step S110), the controller 250 resumes the suspended application (step S114).

### (6) Second specific example

A description of a process in a second specific example will be given below. In the second specific example, the condition for suspension includes the condition that a specific event occurs in addition to the condition that inhalation by the user is detected, and the condition for resumption is the condition that the elapsed time since detection of inhalation performed by the user reaches the first threshold. It is assumed that the first threshold is set to five seconds. It is assumed that the inhaler device 100 is the inhaler device 100A according to the first configuration example and preheating is not necessary.

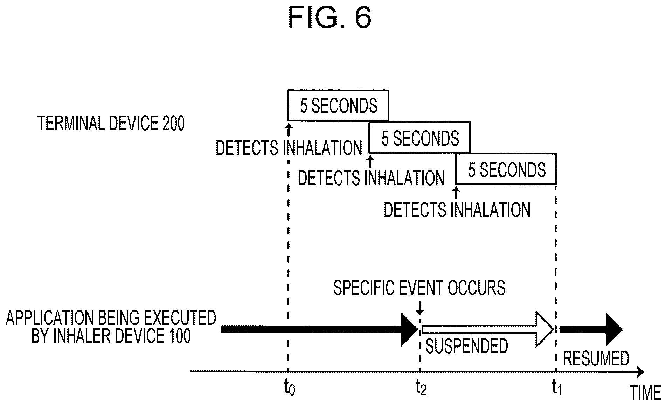

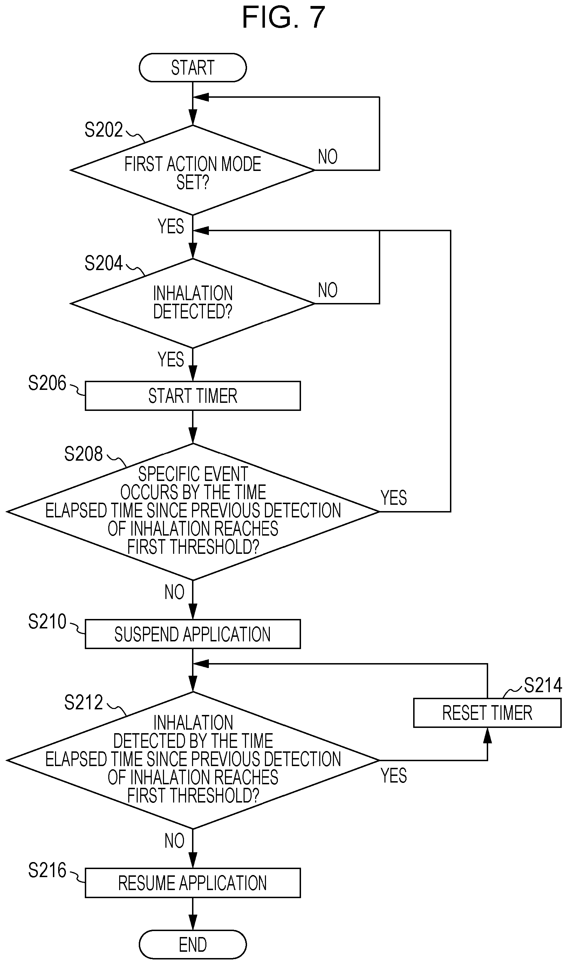

Fig. 6 is a schematic diagram of operations of the terminal device 200 in the second specific example. In the example illustrated in Fig. 6, first inhalation is detected at time to, second inhalation is detected within five seconds of the first inhalation, and third inhalation is detected within five seconds of the second inhalation. Thereafter, no inhalation is detected until time ti five seconds after detection of the third inhalation. Meanwhile, a specific event occurs at time t₂ between the second inhalation and the third inhalation. Therefore, the terminal device 200 suspends an application that is being executed at time t₂ and resumes the application at time ti. A description of the flow of the process in the present specific example will be given below with reference to Fig. 7.

Fig. 7 is a flowchart of an example of the flow of the process, in the second specific example, performed by the terminal device 200 according to the present embodiment. As illustrated in Fig. 7, the controller 250 determines whether the first action mode is set as the action mode of an application that is being executed (step S202). If it is determined that the first action mode is not set (NO in step S202), the process returns to step S202 again.

If it is determined that the first action mode is set (YES in step S202), the controller 250 determines whether inhalation by the user is detected (step S204). If it is determined that inhalation by the user is not detected (NO in step S204), the process returns to step S204 again. If it is determined that inhalation by the user is detected (YES in step S204), the controller 250 starts a timer for measuring the elapsed time (step S206).

Next, the controller 250 determines whether a specific event occurs in the application that is being executed, by the time the elapsed time since the previous detection of inhalation measured by the timer reaches the first threshold (step S208). If it is determined that a specific event does not occur by the time the elapsed time reaches the first threshold, the timer is canceled, and the process returns to step S204 again. On the other hand, if it is determined that a specific event occurs by the time the elapsed time reaches the first threshold, the controller 250 suspends the application that is being executed (step S210).

Next, the controller 250 determines whether inhalation by the user is detected by the time the elapsed time since the previous detection of inhalation measured by the timer reaches the first threshold (step S212). If it is determined that inhalation by the user is detected by the time the elapsed time reaches the first threshold (YES in step S212), the controller 250 resets the timer to zero and measures the elapsed time again (step S214). On the other hand, if it is determined that inhalation by the user is not detected by the time the elapsed time reaches the first threshold (NO in step S212), the controller 250 resumes the suspended application (step S216).

### 3. Supplementary description

Although a preferred embodiment of the present invention has been described in detail above with reference to the attached drawings, the present invention is not limited to the above-described example. It is obvious that one of ordinary skill in the art of the present invention can conceive of various changes or corrections without departing from the technical spirit stated in the claims, and it is understood that such changes and corrections are also within the technical scope of the present invention as a matter of course.

For example, in the above-described embodiment, although an example where the control device according to the present invention is implemented as the terminal device 200 has been described, the present invention is not limited to such an example. In an example, the control device may be implemented as the inhaler device 100. In this case, the inhaler device 100 determines the timings of suspension/resumption on the basis of inhalation information and transmits to the terminal device 200 a signal for giving an instruction for suspending an application that is being executed by the terminal device 200 and a signal for giving an instruction for resuming the application. In another example, the control device may be implemented as a cloud server. In this case, the terminal device 200 transmits inhalation information to the server. The server determines the timings of suspension/resumption on the basis of the inhalation information and transmits to the terminal device 200 a signal for giving an instruction for suspending an application that is being executed by the terminal device 200 and a signal for giving an instruction for resuming the application.

Note that a series of processes performed by the devices described herein may be implemented as any of software, hardware, or a combination of software and hardware. A program that is the software is stored in advance in, for example, a recording medium (non-transitory medium) that is included in each device or that is externally provided. Each program is loaded to a RAM upon execution by a computer and executed by a processor such as a CPU. Examples of the recording medium include a magnetic disc, an optical disc, a magneto-optical disc, and flash memory. In addition, the computer program may be distributed via, for example, a network without using a recording medium.

The processes described herein with reference to the flowcharts and sequence charts need not be performed in the illustrated order. Some process steps may be performed in parallel, an additional process step may be employed, and some process steps may be omitted.

### Reference Signs List

- 1: system
- 100: inhaler device
- 112: sensor
- 115: communicator
- 116: controller
- 150: stick substrate
- 200: terminal device
- 210: sensor
- 220: notifier
- 230: communicator
- 240: memory
- 250: controller

## Claims

1. A control device comprising:
a controller that suspends an application in progress, in response to a condition for suspension related to an inhaler device associated with a terminal device being satisfied while the application is being executed by the terminal device.

2. The control device according to claim 1, wherein the condition for suspension includes a condition that a predetermined user action performed by using the inhaler device is detected.

3. The control device according to claim 2, wherein the predetermined user action is inhalation using the inhaler device.

4. The control device according to claim 2, wherein the predetermined user operation is an operation performed on the inhaler device for enabling inhalation using the inhaler device.

5. The control device according to any one of claims 2 to 4, wherein the condition for suspension further includes a condition that a predetermined event occurs in the application.

6. The control device according to claim 5, wherein the predetermined event includes an event for which an operation is to be performed on the application within a predetermined time from when the event occurs.

7. The control device according to any one of claims 2 to 6, wherein the controller resumes the suspended application in response to a condition for resumption related to the inhaler device being satisfied.

8. The control device according to claim 7, wherein the condition for resumption includes a condition that an elapsed time since detection of the predetermined user action performed by using the inhaler device reaches a first threshold.

9. The control device according to claim 8, wherein the controller sets the predetermined threshold on the basis of a characteristic of a user of the inhaler device.

10. The control device according to claim 8 or 9, wherein the controller sets the predetermined threshold on the basis of a type of the detected predetermined user action.

11. The control device according to any one of claims 8 to 10, wherein the controller sets the predetermined threshold on the basis of the number of times the predetermined user action performed is detected while the application is suspended.

12. The control device according to any one of claims 7 to 11, wherein the condition for resumption includes a condition that the number of times the predetermined user action performed is detected while the application is suspended reaches a second threshold.

13. The control device according to any one of claims 7 to 12, wherein the controller provides to a user of the terminal device, information indicating a timing when the suspended application is to be resumed.

14. The control device according to any one of claims 1 to 13, wherein
the terminal device includes a display unit that displays an output screen of the application, and
the controller controls display by the display unit while the application is suspended, on the basis of a characteristic of the application.

15. The control device according to any one of claims 1 to 14, wherein
for the application, a first action mode in which suspension of the application is allowed or a second action mode in which suspension of the application is not allowed is set, and
the condition for suspension further includes a condition that the first action mode is set for the application.

16. The control device according to any one of claims 1 to 15, wherein the control device is included in the inhaler device.

17. The control device according to any one of claims 1 to 15, wherein the control device is included in the terminal device.

18. A control method comprising:
suspending an application in progress, in response to a condition for suspension related to an inhaler device associated with a terminal device being satisfied while the application is being executed by the terminal device.

19. A program for causing a computer to function as:
a controller that suspends an application in progress, in response to a condition for suspension related to an inhaler device associated with a terminal device being satisfied while the application is being executed by the terminal device.
